# EUROPEAN PATENT APPLICATION

(11) **EP 3 916 006 A1**
(43) Date of publication of application: **01.12.2021**
(21) Application number: 20382443.8
(22) Date of filing: 26.05.2020
(51) Int. Cl.: C07K 14/47, A61K 38/00, A61P 1/00, A61P 3/04, C07K 14/77, C12N 9/36

(54) **PEPTIDES CAPABLE OF INDUCING ANOREXIC HORMONES, COMPOSITIONS AND USES THEREOF**

(71) Applicant: Consejo Superior de Investigaciones Científicas (CSIC), 28006 Madrid (ES)
(72) Inventor: RECIO SÁNCHEZ, Isidra, 28049 Madrid (ES); SANTOS HERNÁNDEZ, Marta, 28049 Madrid (ES); MIRALLES BURAGLIA, Beatriz, 28049 Madrid (ES); HERNÁNDEZ LEDESMA, Blanca, 28049 Madrid (ES); AMIGO GARRIDO, Lourdes, 28049 Madrid (ES)
(74) Representative: Pons

(57) **Abstract**

The invention discloses peptides that are resistant to gastrointestinal digestion and promote the secretion of the hormones cholecystokinin and glucagon-like peptide-1, compositions that comprise said peptides and the uses of the peptides and compositions thereof.

SEQUENCE LISTING

<110> Consejo Superior de Investigaciones Científicas - CSIC

<120> Peptides capable of inducing anorexic hormones, compositions and uses
thereof

<130> EP1641.1563

<160> 20

<170> BiSSAP 1.3.6

<210> 1
<211> 7
<212> PRT
<213> Artificial Sequence

<220>
<223> GLP-1 and CCK secretion inducing peptide

<400> 1
Trp Ile Arg Gly Cys Arg Leu
1               5

<210> 2
<211> 11
<212> PRT
<213> Artificial Sequence

<220>
<223> GLP-1 and CCK secretion inducing peptide

<400> 2
Val Ala Trp Arg Asn Arg Cys Lys Gly Thr Asp
1               5                   10

<210> 3
<211> 9
<212> PRT
<213> Artificial Sequence

<220>
<223> GLP-1 and CCK secretion inducing peptide

<400> 3
Trp Arg Asn Arg Cys Lys Gly Thr Asp
1               5

<210> 4
<211> 4
<212> PRT
<213> Artificial Sequence

<220>
<223> GLP-1 and CCK secretion inducing peptide

<400> 4
Arg Tyr Leu Gly

1

<210> 5
<211> 7
<212> PRT
<213> Artificial Sequence

<220>
<223> GLP-1 and CCK secretion inducing peptide

<400> 5
Arg Tyr Leu Gly Tyr Leu Glu
1               5

<210> 6
<211> 5
<212> PRT
<213> Artificial Sequence

<220>
<223> GLP-1 and CCK secretion inducing peptide

<400> 6
Arg Tyr Leu Gly Tyr
1               5

<210> 7
<211> 5
<212> PRT
<213> Artificial Sequence

<220>
<223> GLP-1 and CCK secretion inducing peptide

<400> 7
Ser Arg Tyr Pro Ser
1               5

<210> 8
<211> 12
<212> PRT
<213> Artificial Sequence

<220>
<223> GLP-1 and CCK secretion inducing peptide

<400> 8
Pro Ala Ala Val Arg Ser Pro Ala Gln Ile Leu Gln
1               5                   10

<210> 9
<211> 7
<212> PRT
<213> Artificial Sequence

<220>
<223> GLP-1 and CCK secretion inducing peptide

<400> 9
Leu Ile Val Thr Gln Thr Met
1               5

<210> 10
<211> 7
<212> PRT
<213> Artificial Sequence

<220>
<223> GLP-1 and CCK secretion inducing peptide

<400> 10
Ala Glu Lys Thr Lys Ile Pro
1               5

<210> 11
<211> 6
<212> PRT
<213> Artificial Sequence

<220>
<223> GLP-1 and CCK secretion inducing peptide

<400> 11
Ile Pro Ala Val Phe Lys
1               5

<210> 12
<211> 10
<212> PRT
<213> Artificial Sequence

<220>
<223> GLP-1 and CCK secretion inducing peptide

<400> 12
Val Leu Leu Pro Asp Glu Val Ser Gly Leu
1               5                   10

<210> 13
<211> 5
<212> PRT
<213> Artificial Sequence

<220>
<223> GLP-1 and CCK secretion inducing peptide

<400> 13
Val Leu Leu Pro Asp
1               5

<210> 14
<211> 9
<212> PRT
<213> Artificial Sequence

<220>

<223> GLP-1 and CCK secretion inducing peptide

<400> 14
Arg Val Ala Ser Met Ala Ser Glu Lys
1               5

<210> 15
<211> 20
<212> DNA
<213> Artificial Sequence

<220>
<223> Primer 1 forward CCK

<400> 15
ccaatttttc ctgcccgcat                                                   20

<210> 16
<211> 21
<212> DNA
<213> Artificial Sequence

<220>
<223> Primer 2 reverse CCK

<400> 16
agaaggagca gtcaagccaa a                                                 21

<210> 17
<211> 20
<212> DNA
<213> Artificial Sequence

<220>
<223> Primer 1 forward GLP-1

<400> 17
agagacatgc tgaagggacc                                                   20

<210> 18
<211> 20
<212> DNA
<213> Artificial Sequence

<220>
<223> Primer 4 reverse GLP-1

<400> 18
ctttcaccag ccacgcaatg                                                   20

<210> 19
<211> 20
<212> DNA
<213> Artificial Sequence

<220>
<223> Primer 5 forward B-actin

<400> 19
agctgcgttt tacacccttt                                                    20


<210> 20
<211> 20
<212> DNA
<213> Artificial Sequence


<220>
<223> Primer 6 reverse B-actin

<400> 20
aagccatgcc aatgttgtct                                                    20

## Description

The invention relates to peptides that promote the secretion of anorexic hormones, compositions that comprise said peptides and the uses of the peptides and compositions thereof. The invention, therefore, relates to the field of health and medicine, specifically medicinal preparations that comprise peptides, and organic chemistry.

### BACKGROUND ART

Obesity and type 2 diabetes are epidemic in both developing and developed countries, and consumption of high-fat diets is thought to be a contributing factor. In particular, consumption of a high-fat diet promotes excess energy intake and contributes to the development of obesity and insulin resistance and to abnormalities of fat metabolism. During gastrointestinal digestion of food, proteins are hydrolyzed into a large variety of peptides and free amino acids. These protein digestion-derived products can act, among other nutrients, as pre-absorptive signaling molecules, by inducing the release of hormones relevant to satiety by gastrointestinal enteroendocrine cells (Fromentin, G. et al., Nutrition Research Reviews, 2012, 25(1), pp. 29-39). Enteroendocrine cells are specialized cells capable of sensing luminal contents due to the existence of nutrient-specific receptors on their apical side. These cells, which reside scattered throughout the intestinal epithelium, produce and release a variety of hormonal regulators, such as I-cells producing cholecystokinin (CCK) or L-cells secreting glucagon-like peptide-1 (GLP-1), that modulate a variety of physiological gastrointestinal and homeostatic functions.

GLP-1 stimulates insulin secretion in response to oral glucose. Besides its insulinotropic activity, GLP-1 also suppresses glucagon, retards gastric emptying, reduces appetite and food intake and can inhibit beat-cell apoptosis and promote beta-cell apoptosis and promote beta-cell regeneration and neogenesis (Seino S. et al., Incretins and regulation of insulin secretion. In Pancreatic Beta Cell in Health and Disease. Seino S, Bell GI, Eds. Tokyo, Japan, Springer, 2008, p. 335-377).

Similarly to GLP-1, CCK also has several physiological effects, namely it stimulates gastric acid, gallbladder and pancreatic secretion, slows gastric emptying, increases the sensation of short-term fullness, modulates gastrointestinal motility and suppresses energy intake (Little et al., Obesity Reviews, 2005, 6, pp. 297-306). Human studies have found that CCK infusion lowers postprandial plasma glucose levels in humans, and this effect is likely to be mediated by its inhibitory effect on gastric emptying or potential insulinotropic actions (Ahren et a/., Journal of Clinical Endocrinology and Metabolism, 2000, 85 1043-1048; Martin et al. Journal of Endocrinology, 2020, 244(1), pp. R1-R15). It was reported that CCK promotes insulin secretion in response to amino acids (Rushakoff, R. J., et al., Journal of Clinical Endocrinology and Metabolism, 1987, Vol. 65 (3), pp. 395-401). Therefore, both GLP-1 and CCK can be exploited to treat excess weight or obesity and type 2 diabetes and other diseases associated with obesity and type 2 diabetes.

Most works have so far concentrated in targeting either GLP-1 or CCK in order to treat the aforementioned pathologies. Many developed products, specially directed at GLP-1, have tried to mimic the hormone and its effects. Other studies have tried to promote the secretion of GLP-1 through peptides originated in food. The document EP3590519 discloses peptides and compositions that contain said peptides that promote the secretion of GLP-1. The document US2012108508 discloses a method for promoting the secretion of GLP-1 comprising the administration of purified κ-casein to a subject. The document JP2004010569 discloses peptides which contain arginine and food compositions that contain said peptides, for the promotion of CCK secretion. Likewise, the document WO2017150536 discloses peptides which have a CCK secretion promoting activity.

Despite such developments, the treatment of obesity and type 2 diabetes and associated pathologies lacks effectivity partly due to individual targeting of hormones GLP-1 or CCK, partly due to the fact that the peptides used are synthetic and non-resistant to the gastrointestinal digestive enzymes. Therefore, there is in the state of the art the necessity of providing with alternative peptides that are from natural origin, resistant to digestive enzymes and capable of targeting both GLP-1 and CCK hormones.

### DESCRIPTION OF THE INVENTION

The inventors discovered that the peptides obtained by enzymatic hydrolysis with food grade enzymes from common food sources like egg white and milk, are resistant to the enzymatic digestion in the gastrointestinal track, allowing them to be orally administered without being degraded by the action and the juices of the digestive track. Furthermore, careful analysis of the peptides obtained revealed that some of these peptides obtained from the enzymatic hydrolysis of egg white and milk have the ability to promote secretion from both cholecystokinin (CCK) and glucagon-like peptide-1 (GLP-1) and as such, are more effective in the treatment of obesity and related diseases like diabetes Type 2.

As such the present invention discloses food related peptides, and compositions comprising said peptides, obtained through enzymatic hydrolysis with food grade enzymes. This makes the peptides obtained resistant to digestive enzymes and able to be administrated via oral. Furthermore, the peptides here disclosed promote the secretion of CCK and GLP-1.

### Peptides and compositions

Therefore, a first aspect of the present invention relates to a peptide resistant to gastrointestinal digestion and capable of inducing anorexic hormone secretion, from here onwards the "peptides of the invention", comprising, or consisting of, an amino acid sequence selected from sequences SEQ ID NO: 1 to 14 and ³⁶⁴PFL³⁶⁶.

The term "peptide" as used herein refers to any amino acid molecule comprising two or more amino acids joined to each other by peptide bonds or modified peptide bonds, i.e. peptide isosteres. Peptides may contain amino acids other than the 20 gene-encoded amino acids. Peptides include amino acid sequences modified either by natural processes, such as post-translational processing, or by chemical modification techniques which are well known in the art. Modifications can occur anywhere in a peptide, including the peptide backbone, the amino acid side-chains and the amino or carboxyl termini. The same type of modification may be present in the same or varying degrees at several sites in a given peptide. Also, a given peptide may contain many types of modifications.

The expression "resistant to gastrointestinal digestion" as used herein refers to the property of the peptides of the invention not being broken down or degraded either by the physic forces applied during the passage through the digestive track (mouth, esophagus, stomach and intestines) like chewing, squeezing and mixing or by the digestive juices to which they come in contact with in the digestive track, namely stomach acid, digestive enzymes (e.g. lipases, amylases, proteases, etc.) and bile.

The expression "capable of inducing anorexic hormone secretion" as used herein refers to the property of the peptides of the invention of inducing, provoking or causing the enteroendocrine cells that are scattered throughout the intestinal epithelium and sense luminal content, to secrete hormones that have an anorexic effect, i.e. lead to a loss of appetite. In a preferred embodiment of the peptides of the invention the anorexic hormone secretion induced is CCK and/or GLP-1. Example of an assay to determine if a sample is capable of inducing anorexic hormone secretion is described in Santos-Hernández et al., Food & Function, 2018, Vol. 9(9), pp. 4702-4713. In the present invention, the inventors discovered that the peptides that present the capacity of inducing GLP-1 secretion are characterized by a net positive charge (isoelectric point ≥ 6) and a hydrophobic character, comprising arginine and an aromatic residue (W or Y) within their sequences.

The terms "net positive charge" and "isoelectric point" as used herein refer to a measure of the pH of a solution in which a protein carries no net charge. When a protein is found at a pH equivalent to its isoelectric point, it will carry globally neutral net electric charge. Proteins that have an isoelectric point lower than the pH of its solution will carry a net negative charge. Likewise, proteins that have an isoelectric point higher than the pH of its solution will carry a net positive charge.

The term "hydrophobic character" as used herein refers to the presence in the peptides of the invention of amino acids having an uncharged, nonpolar side chain that is relatively insoluble in water. Examples of naturally occurring hydrophobic amino acids are alanine (A), leucine (L), isoleucine (I), valine (V), proline (P), phenylalanine (F), tryptophan (W), and methionine (M).

The term "aromatic residue" refers to amino acids that contain an aromatic ring such as phenylalanine, tyrosine, tryptophan and histidine.

CCK, also called pancreozymin, and GLP-1 are peptide hormones of the gastrointestinal system responsible for stimulation the digestion of fat and proteins. The latter hormone is also an incretin, i.e., a metabolic hormone that stimulate a decrease in blood glucose levels. Incretins are released after eating and augment the secretion of insulin released from pancreatic beta cells.

The peptides of the invention may be used for the formulation of compositions which may comprise other components together with the peptides of the invention. As such, another aspect of the invention is a composition, from here onwards the "composition of the invention", comprising one or more of the peptides of the invention. In preferred embodiment the composition of the invention is a food, a beverage or a pharmaceutical composition, from here onwards the "pharmaceutical composition of the invention".

Examples of foods that may comprise the peptides of the invention are, without limitation, animal feed, dairy products, vegetable products, meat products, snacks, chocolates, drinks, baby food, cereals, fried foods, bakery products, cookies, etc. Examples of dairy products include, but are not limited to, fermented milk products (such as, but not limited to, yogurt or cheese) or non-fermented products (for example, but not limited to, cream, butter, margarine or whey). A vegetable product is, for example, but not limited to, a cereal in any form of presentation, fermented or unfermented, or a snack. However, in a particular embodiment, the food product is selected from the group consisting of a dairy product, a meat product, a vegetable product and an animal feed.

Examples of beverages are, without limitation, juice beverages (e.g., beverages comprising one or more fruit juices and/or one or more vegetable juices), hydration beverages such as those with added electrolytes, frozen or chilled beverages, caffeinated beverages, carbonated beverages, non-carbonated beverages, zero to low calorie drinks (for example, 0 - 150 kcals and up to 10 grams sugar), such as diet or other reduced calorie beverages, and syrups or concentrates. Non-limiting examples of suitable dairy-containing beverages include milk (e.g., 2% milk) and other beverages containing milk (e.g., coffee drinks containing milk). In some embodiments, the food product is a beverage that may require refrigeration. In some embodiments, the food product is a beverage that is stable and safe to store at ambient conditions not requiring refrigeration. In some embodiments, the food product comprises a low pH of less than about 4.5. Suitable beverages may comprise sweetener, water, dairy, caffeine, carbonation, fruit juice, vegetable juice, food grade acid, or a mixture thereof. Beverage products disclosed herein include ready-to-drink liquid formulations (i.e., ready-to-drink beverages), beverage concentrates, beverage pods, and the like. The term "ready-to-drink" refers to a beverage formulated to be ingested as-is. Thus, in some embodiments, the ready-to-drink beverage requires no dilution or additions prior to ingestion by a consumer.

In a preferred embodiment, the pharmaceutical composition of the invention further comprises an acceptable pharmaceutical adjuvant, an acceptable pharmaceutical vehicle and/or another active ingredient.

The expression "acceptable pharmaceutical adjuvant", as used herein refers to a substance which assists in the absorption of the elements of the pharmaceutical composition of the invention, stabilizes such elements, activates or assists in the preparation of the composition in the sense of giving it consistency or providing flavors that make it more pleasant. Thus, the excipients or adjuvants could have the function of keeping the ingredients together, as for example in the case of starches, sugars or celluloses, the function of sweetening, the function of coloring, the function of protecting the composition, as for example to insulate it from air and/or humidity, the function of filling a tablet, capsule or any other form of presentation, a disintegrating function to facilitate the dissolution of the components and their absorption in the intestine, without excluding other types of excipients not mentioned in this paragraph.

The expression "acceptable pharmaceutical vehicle", as used herein refers to a substance used in the composition to dilute any of its constituent parts to a certain volume or weight. The pharmaceutically acceptable vehicle is a substance that is inert or has an identical action to any of the elements included in the pharmaceutical composition of the invention. The function of the vehicle is to facilitate the incorporation of other elements, to allow a better dosage and administration or to give consistency and form to the composition.

The term "active ingredient" refers to a therapeutically active compound, as well as to any prodrugs thereof and the pharmaceutically acceptable salts, hydrates and solvates of the compound and the prodrugs.

The composition of the present invention may also take the form of a sustained release drug formulation or any other conventional release system, such as nanoparticles, liposomes or nanospheres, polymeric material, biodegradable or non-biodegradable implant or biodegradable microparticles, such as biodegradable microspheres. This composition and/or its formulations can be administered to a mammal subject, and therefore to humans, in various ways, including intraperitoneal, intravenous, intrastromal, intra-arterial, surgical implant, internal surgical paint or infusion pump.

The administration of the pharmaceutical composition of the invention shall be carried out in a therapeutically effective dose, which is sufficient to demonstrate a benefit for the patient. Such a benefit may involve the improvement of at least one symptom related to the disease or pathology that affects the patient. The prescription of the treatment, that is to say, the decisions about the doses, periodicity, duration, etc., will fall under the responsibility of the general practitioner or the specialist who attends the affected patient.

The term "therapeutically effective dose" or "therapeutically effective amount", as used in the present invention, refers to an amount (or amount per unit mass of the individual to whom it is administered) of a drug or therapeutic agent that causes a detectable therapeutic effect on an individual or a group of individuals to whom it is administered, causing minimal side effects or toxicity. The therapeutically effective amount useful to produce a therapeutic effect can be determined by conventional techniques well known to specialists in the technique. The term "therapeutically effective dosage-50" or "therapeutically effective dosage-95" includes a statistical value in which the therapeutic effect must be detectable in 50% or 95% of the individuals to whom it is administered. With regard to the toxic effects of the drug or compound, it is preferable that the effective therapeutic dose does not cause any. However, although toxic effects may sometimes occur, a compromise may be reached in which these are considered preferable to the normal development of the disease or pathology without the administration of the drug or compound and may in turn be treated by additional therapies.

### Uses of the peptides or compositions of the invention

As mentioned previously the peptides of the invention lead to the secretion of the CCK and GLP-1 hormones, being useful for treating obesity, type-2 diabetes, and associated diseases. Therefore, an aspect of the present invention relates to the peptides of the invention or the composition of the invention for use as a medicament.

The term "medicament" as used herein refers to any substance or combination of substances which has properties for treating or preventing disease in organisms, preferably humans, or which may be used in or administered to organisms, preferably humans, with a view to restoring, correcting or modifying physiological functions by exerting a pharmacological, immunological or metabolic action. The use of the peptides of the invention or the composition of the invention as a medicament may be carried out alone or in combination with other medicinal products or compositions as a combination therapy and may be administered simultaneously or at different time intervals (sequentially).

Another aspect of the invention relates to the peptides of the invention or the composition of the invention for use in the induction of cholecystokinin secretion and/or glucagon-like Peptide 1 secretion in a mammal, preferably a human.

The expression "induction of cholecystokinin secretion and/or glucagon-like Peptide 1 secretion" as used herein refers to the property of the peptides of the invention of inducing, provoking or causing the enteroendocrine cells that are scattered throughout the intestinal epithelium and sense luminal content, to secrete CCK and/or GLP-1.

Since CCK and GLP-1 hormones induce the increase in insulin secretion from the pancreatic beat cells another aspect of the present invention relates to the peptides of the invention or the composition of the invention for use in the increase of post-prandial insulin secretion in a mammal, preferably a human.

The level of insulin in the blood of an individual can be measured at different time points after the ingestion of food or, to be comparable between individuals, after a 75 g oral dose glucose load. In healthy subjects the insulin level will vary within a range, being that at 30 minutes after glucose load the normal insulin blood level will be of less than 174 pmol\L, at 1 hour after glucose load the normal insulin blood level will range between 125 and 1597 pmol\L, at 2 hours after glucose load the normal insulin blood level will range between 111 and 1153 pmol\L, and at 3 hours after glucose load the normal insulin level will be again less than 174 pmol\L. Therefore, the ideal time for comparing insulin levels is at 1 and 2 hours after ingestion of food or glucose load. As such, the expression "increase of post-prandial insulin secretion" refers to the augment or raise of the insulin levels in the blood in an individual at 1 hour or 2 hours after food ingestion or glucose load ingestion accompanied with the peptides of the invention or composition of the invention, compared to the insulin levels of the blood in the same individual 1 hour or 2 hours after a similar food ingestion or glucose load without the peptides of the invention or the composition of the invention.

As it is well documented, the increase in insulin levels in the blood leads to a lowering of the glucose levels in the blood. Therefore, another aspect of the invention relates to the peptides of the invention or the composition of the invention for use in lowering plasma blood glucose levels in a mammal, preferably a human. Another aspect of the present invention relates to peptides of the invention or the composition of the invention for use in treating and/or preventing hyperglycemia in a mammal, preferably a human.

The International Diabetes Federation provides as a general guidance that the blood glucose levels in a healthy individual are between 4.0 to 5.4 mmol\L when fasting and up to 7.8 mmol\L 2 hours after eating. For individuals that suffer from diabetes the UK National Institute for Clinical Excellence recommends the following ranges to be maintained: for individuals with Type 2 diabetes the levels should be of 4 to 7 mmol\L before meals and under 8.5 mmol\L 2 hours after meals; for individuals with Type 1 diabetes the levels should be of 5 to 7 mmol\L upon waking up, of 4 to 7 mmol\L before meals and of 5 to 9 mmol\L 2 hours after meals; and for children with Type 1 diabetes the levels should be of 4 to 7 mmol\L upon walking up, of 4 to 7 mmol\L before meals and of 5 to 9 mmol\L 2 hours after meals. Several tests to determine the glucose blood levels are commonly available, being the most common the prick test where the subject is pricked to obtain a drop of blood which is then applied to a test strip which is then inserter into a blood glucose monitor which will readout the blood glucose level.

As such, the expression "lowering plasma blood glucose levels in a mammal" as used herein relates to the reduction of the plasma blood glucose levels or concentration in a subject regardless if the levels are within or outside the recommended parameters, as detailed in the previous paragraph, for healthy and diabetic subjects, due to the ingestion or administration of the peptide or composition of the invention which leads to the production of insulin due to the secretion of the integrin hormones CCK and GLP-1. Such lowering or reduction of the glucose blood levels is in relation to the comparison of the subject blood glucose levels before and after ingestion or administration of the peptide or composition of the invention. Such comparison can be made in different ways: the levels of blood glucose of a subject 2 hours after taking the peptide of the invention or composition of the invention compared to the subject blood glucose levels before meals; the levels of blood glucose of a subject 2 hours after taking the peptide of the invention or composition of the invention together with food compared to the subjects' blood glucose levels 2 hours after ingesting the same food without the peptide or composition of the invention.

The term "treatment" as used herein refers to fighting the effects caused as a consequence of the disease or pathological condition of interest (like hyperglycemia) in a subject (preferably a mammal and, more preferably, a human), including:
i) Inhibiting the disease or pathological condition, i.e., stopping its development;
ii) Alleviating the disease or pathological condition, i.e. causing the regression of the disease or pathological condition or its symptoms;
iii) Stabilizing the disease or pathological state.

The term "prevention", as herein refers to the avoiding the appearance or reappearance of the condition, that is, avoiding the pathological state of the disease in a subject (preferably a mammal and, more preferably, a human), in particular, when said subject has a predisposition to the pathological condition but has not yet been diagnosed.

The expression "in treating and/or preventing hyperglycemia" as used herein relates to the reduction or prevention or rising of the plasma blood glucose levels or concentration in a subject whose blood levels are above or will be above the recommended ones, as stated above, preferably wherein the subject suffers from a form of diabetes (Type 1 or Type 2). Such lowering or reduction of the glucose blood levels is in relation to the comparison of the subject blood glucose levels before and after ingestion or administration of the peptide or composition of the invention. Such comparison can be made in different ways: the levels of blood glucose of a subject 2 hours after taking the peptide of the invention or composition of the invention compared to the subject blood glucose levels before meals or upon waking up; the levels of blood glucose of a subject 2 hours after taking the peptide of the invention or composition of the invention together with food compared to the subjects' blood glucose levels 2 hours after ingesting the same food without the peptide or composition of the invention.

Another aspect of the invention relates to the peptide of the invention or the composition of the invention for use in regulating glucose homeostasis in a mammal, preferably in a human.

The term "glucose homeostasis" as used herein refers to the maintenance of adequate glucose blood levels, which is necessary for survival. Normal glucose homeostasis is primarily maintained by two hormones with counter effects: glucagon and insulin. Glucagon promotes the raise of glucose blood levels in the blood while insulin promotes the decrease of glucose blood levels.

The expression "regulating glucose homeostasis" as used herein refers to ensuring the maintenance of adequate glucose blood levels, as previously mentioned, in a subject by stopping or preventing the increase of the glucose blood levels by the ingestion or administration of the peptides or the composition of the invention.

As is known in the field, both hormones, CCK and GLP-1 reduce the appetite and food intake. Therefore, another aspect of the present invention relates to the peptides of the invention or the composition of the invention for use in treatment or prevention of obesity or overweight, and related diseases, preferably diabetes, in a mammal, preferably a human.

The terms "treatment" was previously defined in this description and such definition is valid for the present aspect of the invention in relation to obesity and overweight.

The term "prevention" as used herein means inhibition of the occurrence of the obesity (body weight gain), namely, ingestion or administration of the peptides of the invention or composition of the invention before the onset of the obesity condition. This means use of the peptides of the invention or composition of the invention as a preventive drug to thereby prevent body weight gain, or to thereby prevent the disease which may be induced by the body weight gain.

The term "obesity" in general refers to an abnormal or excessive fat accumulation that presents a risk to health, being "overweight" a level of obesity. A crude population measure of obesity in adults is the body mass index (BMI), a person's weight (in kilograms) divided by the square of his or her height (in meters). The term "obesity" is herein adopted to describe a condition characterized by, preferably, a BMI ≥ 30 kg/m2 while the term "overweight" is herein adopted to describe a condition characterized by, preferably, a BMI ≥ 25 kg/m² but < 30 kg/m². For adolescents, "obesity" refers to a condition characterized by two standard deviations body mass index for age and sex from the World Health Organization (WHO) growth reference for school-aged children and adolescents. The terms "obesity" and "overweight" thus imply a medical indication for treatment.

The terms "related or associated diseases/disorders" and "diseases/ disorders caused by overweight and/or obesity" as used herein comprise: metabolic syndrome, hypertension, hyperglycaemia, inflammation, type-2 diabetes, cardiovascular disease, hypercholesterolemia, hormonal disorders, infertility, etc.

Another aspect of the present invention relates to the peptides of the invention or the composition of the invention for use in reducing high fat diet or obesity induced inflammation in a mammal, preferably a human.

The expression "high fat diet or obesity induced inflammation" refers to the low grade inflammation characterized by the over production of pro-inflammatory adipocytokines, said inflammation being present in individuals that are overweight or obese. The level of reduction of inflammation can be determined by comparing the level of blood markers of inflammation before and after a course of ingestion or administration of the peptides or the composition of the invention. Examples of blood markers of inflammation are the erythrocyte sedimentation rate (ESR), the C-reactive protein level and plasma viscosity. Such tests are commonly used to detect increased protein in the blood, a marker of inflammation. The course of ingestion or administration of the peptides or the composition of the invention refers to a period of time during which is necessary to ingest or administer the peptides or composition of the invention so that a reduction of high fat diet or obesity induced inflammation is appreciable.

A typical disease or disorder associated with long-term high fat diet or obesity is hepatic steatosis, the accumulation of excess fat in the liver. Therefore, another aspect of the present invention relates to the peptides of the invention or the compositions of the invention for use in lowering liver fat content in a mammal, preferably a human.

The expression "lowering liver fat content" as used herein refers to the reduction or decrease in the amount of fat deposited in the liver. Such lowering or reduction can be detected by a reduction of the enlargement of the liver due to a decrease in the inflammation cause by the excess fat deposited. Other methods to detect liver fat excess, and therefore also detect a reduction of liver fat excess, are blood tests for liver enzymes like the alanine aminotransferase and aspartate aminotransferase, imaging studies like ultrasound exam, computer tomography abdominal scan, magnetic resonance imaging scan, vibration-controlled transient elastography scan, and a liver biopsy. The comparison of the results of one or more of the mentioned tests or exams before and after a course of ingestion or administration, as defined previously, will determined the level or lowering of the liver fat content.

In another aspect, the present invention relates to a kit, from here onwards the "kit of the invention" comprising the peptides of the invention or the composition of the invention. In addition to the peptides of the invention or the composition of the invention, the kit may comprise other components useful in the implementation of the present invention, such as, delivery vehicles, material holders, etc. In addition to the above components, the kits may also include instructions for practicing the object of the invention. These instructions may be present in the above-mentioned kits in a variety of forms, one or more of which may be present in the kit. One way in which these instructions may be present is as information printed on a suitable medium or substrate, e.g., a sheet or sheets of paper on which the information is printed, on the kit packaging, on a package insert, etc. Another medium would be a computer-readable medium, e.g. CD, USB, etc., on which the information has been recorded. Another means that may be present is a website address that can be used over the Internet to access information at a remote site. Any convenient means can be present in the kits.

All uses related to the peptides of the invention or the composition of the invention are also applicable as uses of the kit. Therefore, another aspect of the invention relates to the kit of the invention for use as a medicament.

Another aspect of the invention relates to the kit of the invention for use in the induction of cholecystokinin secretion and/or glucagon-like peptide 1 secretion in a mammal.

Another aspect of the invention relates to the kit of the invention for use in the increase of post-prandial insulin secretion in a mammal.

Another aspect of the invention relates to the kit of the invention for use in lowering plasma blood glucose levels in a mammal.

Another aspect of the invention relates to the kit of the invention for use in lowering liver fat content in a mammal.

Another aspect of the invention relates to the kit of the invention for use in reducing high fat diet or obesity induced inflammation in a mammal.

Another aspect of the invention relates to the kit of the invention for use in regulating glucose homeostasis in a mammal.

Another aspect of the invention relates to the kit of the invention for use in treating and/or preventing hyperglycemia in a mammal.

Another aspect of the invention relates to the kit of the invention for use in treatment or prevention of obesity or overweight in a mammal.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

### DESCRIPTION OF THE DRAWINGS

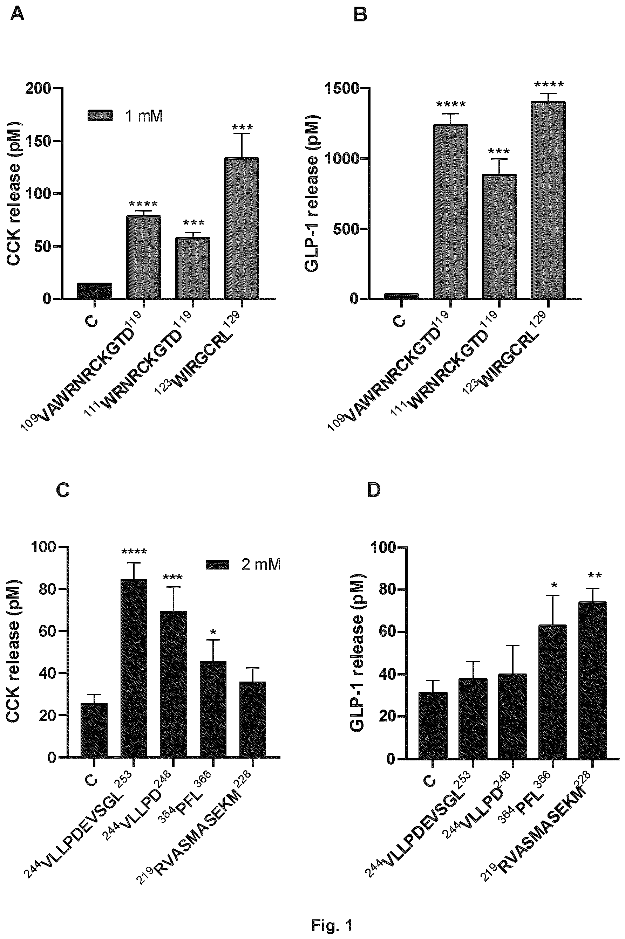

**Fig. 1****.** CCK (A) and GLP-1 (B) secretion after 2 h incubation of STC-1 cells with synthetic peptides derived from egg white lysozyme at 1 mM. CCK (C) and GLP-1 (D) secretion after 2 h incubation of STC-1 cells with synthetic peptides derived from egg white ovalbumin at 2 mM. Hormonal secretion was determined by ELISA. Error bars indicate SEM (n=3). Statistical significance compared with control (t Student) is indicated by ****P < 0.0001, ***P < 0.001, **P < 0.01 and *P < 0.05.

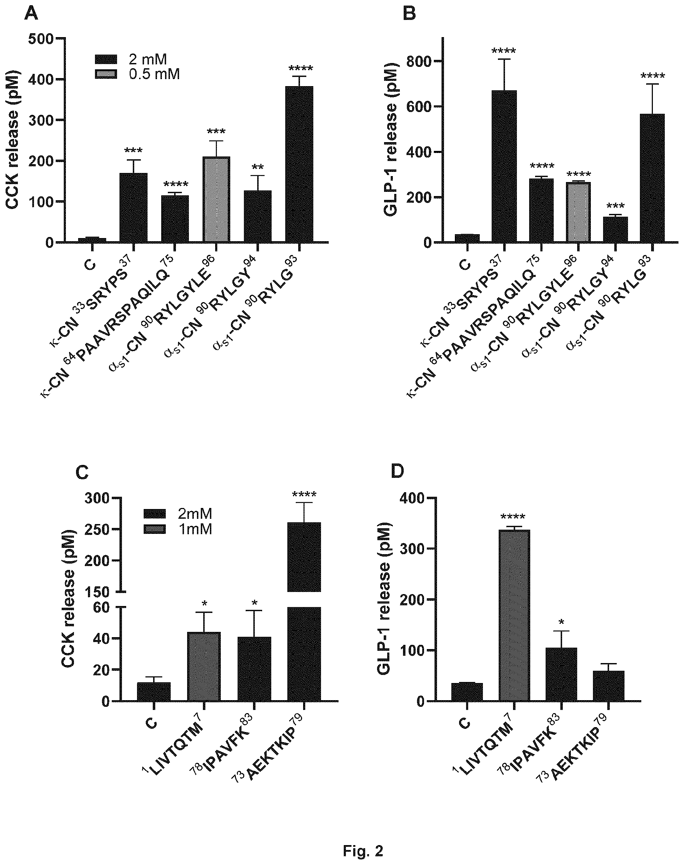

**Fig. 2****.** CCK (A) and GLP-1 (B) secretion after 2 h incubation of STC-1 cells with synthetic peptides derived from milk casein (CN), κ-CN and αₛ₁-CN, at 2 mM and 0.5 mM. CCK (C) and GLP-1 (D) secretion after 2 h incubation of STC-1 cells with synthetic peptides derived from milk β-lactoglobulin at 2 mM and 1mM. CCK and GLP-1 secretion was determined by ELISA. Error bars indicate SEM (n=3). Statistical significance compared with control (t Student) is indicated by ****P < 0.0001, ***P < 0.001, **P < 0.01 and *P < 0.05.

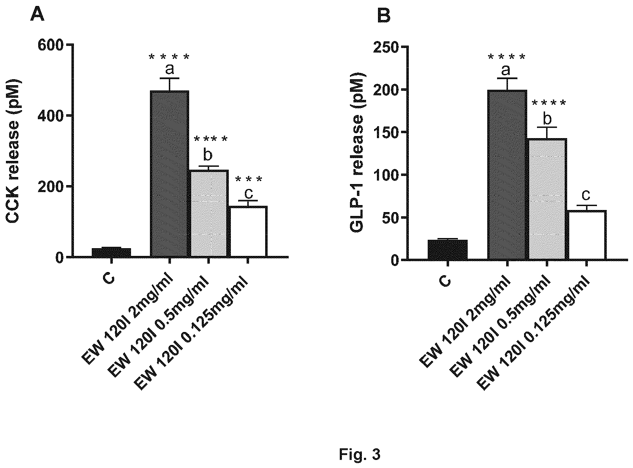

**Fig. 3****.** CCK (A) and GLP-1 (B) secretion after 2 h incubation of STC-1 cells with egg white *in vitro* gastrointestinal digests at 120 min of intestinal phase (EW 1201), at different protein concentrations (2, 0.5 and 0.125 mg/mL). CCK and GLP-1 secretion was determined by ELISA. Error bars indicate SEM (n=3). Statistical significance compared with control (one-way ANOVA with Tukey post hoc test) is indicated by ****P < 0.0001 and ***P < 0.001. Different letters denote statistically significant differences (P < 0.05) between different protein concentrations.

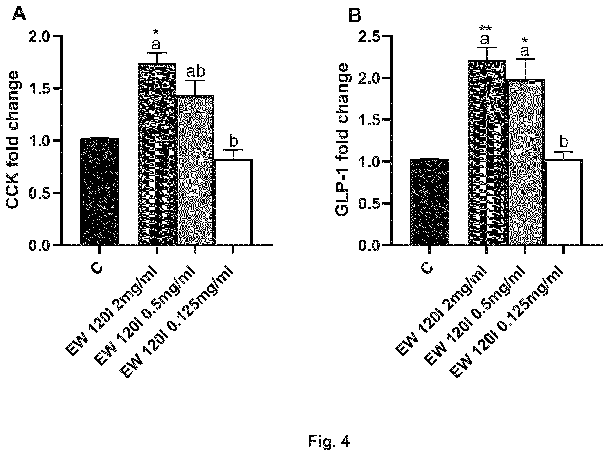

**Fig. 4****.** *Cck* (A) and *Glp-1* (B) mRNA levels in STC-1 cells after 2 h incubation with *in vitro* gastrointestinal digests of egg white proteins at different protein concentrations (2, 0.5 and 0125 mg/mL). EW 1201 corresponds to 120 min after gastric digestion following 120 min of intestinal phase. Error bars indicate SEM (n=3). Statistical significance compared with control (Krustal-Wallis test) is indicated by **P < 0.01 and *P < 0.05. Different letters denote statistically significant differences (P < 0.05) between different protein concentrations

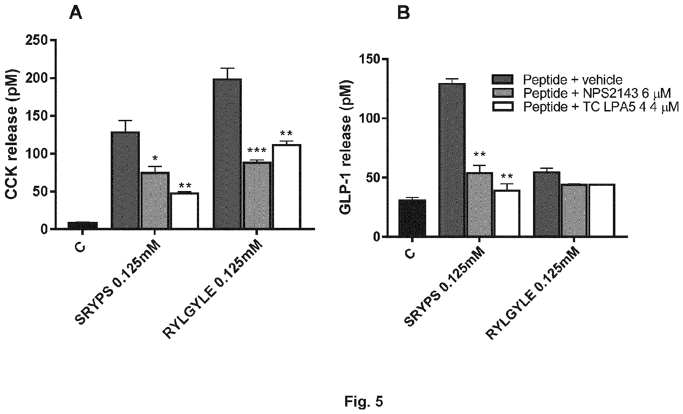

**Fig. 5****.** CCK (A) and GLP-1 (B) secretion with synthetic peptides and the inhibitors NPS2143 at 6 µM or TC LPA5 4 at 4 µM, or vehicle (0.1% DMSO). Peptides were 1 h incubated at 0.125 mM in STC-1 cell line after 20 min incubation of inhibitors or vehicle thereof. CCK and GLP-1 secretion was determined by ELISA. Error bars indicate SEM (n=3). Statistical significance compared with each peptide + vehicle (one-way ANOVA with Tukey post hoc test) is indicated by ***P < 0.001, **P < 0.01 and *P < 0.05.

### Examples

### a) Materials and methods

### a. 1. Samples

Egg white was manually separated from ecological fresh eggs obtained in a local supermarket. Synthetic peptides derived from egg and from milk proteins were either purchased from CSBio Ltd (Shanghai, China) or synthesized in house using conventional solid-phase FMOC synthesis with a 433A peptide synthesizer (Applied Biosystems, Warrington, UK). Their purity (> 90%) was verified in our laboratory by reverse phase high performance liquid chromatography and tandem mass spectrometry (HPLC-MS/MS). The protein content of the synthetic peptides and digests was determined by elemental analysis.

### a.2. In vitro simulated gastrointestinal digestion

Egg white, casein or whey protein was digested according to the INFOGEST *in vitro* gastrointestinal digestion protocol (Brodkorb et al., Nature 2019 Vol. 14, pp. 991-1014). The protein in the oral phase was 30 mg of protein/mL, and then 1:1 (v:v) diluted with simulated salivary fluid without amylase for the absence of starch in the sample. The intestinal phase was conducted by adding pancreatin from porcine pancreas (100 U trypsin activity/mL of a final mixture, Sigma-Aldrich) and porcine bile extract (B8631-100G, Sigma-Aldrich) in simulated intestinal fluid. Because of the cytotoxic effects of 10 mM bile salts in the cell line STC-1, their concentration was reduced to 2.5 mM in the intestinal phase (Santos-Hernandez et al., 2018).

Intestinal digests were centrifuged for 15 min at 5,000 x g to separate soluble and insoluble material, followed by snap freezing in liquid nitrogen. The supernatants were freeze-dried, and the protein content was determined by elemental analysis.

### a.3. Cell culture experiments

STC-1 cells, supplied by ATCC (ATCC CRL3254), were cultured in Dulbecco's modified Eagle's medium containing 4.5 g/L of glucose and 5 mM L-glutamine (DMEM, Life Technologies, Paisley, UK) supplemented with 100 U/mL penicillin, 100 mg/L streptomycin, amphotericin, and 10% foetal bovine serum. STC-1 cells were incubated at 37°C in a 5% CO₂ humidified atmosphere and when they reached 80% confluence, they were trypsinized and seeded according to each cell study requirement. The cells were used between passage numbers 25 - 40.

### a.3.1. Cell viability

A concentration of 5 × 10⁴ cells per well were seeded into 96-well plates and cultured for 24 h at 37°C in a 5% CO₂ humidified atmosphere. After 2.5 h incubation with protein digests, and synthetic peptides at different concentrations (2 mg/mL in protein digests and from 2 mM to 1 mM in synthetic peptides), the medium was removed and the plate was further incubated (37°C for 1 h) with Alamar Blue Cell Viability Reagent (Thermo Fisher Scientific) (100 µL, diluted 1:10 v:v), prior to a fluorescence reading (excitation wavelength of 580 nm and fluorescence emission at 610 nm) in a microplate reader. The inhibitors NPS2143 (inhibitor of Calcium sensing receptor, SML0362, Sigma-Aldrich) and TC LPA5 4 (inhibitor of GPR93, 4708/10, Tocris Bioscience) were also incubated at different concentrations for 1.5 h in HEPES buffer prior to incubation with Alamar Blue Reagent.

### a.3.2. Hormone secretion and gene expression studies

Cells were cultured at 37 °C in a 5% CO₂ humidified atmosphere for 48 h into 24-well plates at a density of 3 × 10⁵ cells per well. Cells were washed twice with HEPES buffer (20 mM HEPES 1 M, 10 mM glucose, 140 mM NaCl, 4.5 mM KCl, 1.2 mM CaCI2, 1.2 mM MgCl₂, pH 7.4) and were incubated for 1 h in HEPES buffer prior to adding buffer (control) or buffer supplemented with protein digests or synthetic peptides. Protein digests were tested at 2, 0.5 and 0.125 mg of protein/mL and synthetic peptides from 2 mM to 0.125 mM. After 2 h incubation, supernatants were collected and stored at -80 °C with Halt Protease and phosphatase inhibitor (Thermo Fisher Scientific, Waltham, MA, USA). Measurement of the CCK and GLP-1 concentration was performed using a commercial enzyme immunoassay CCK 26-33, non-sulphated EIA Kit (Phoenix Pharmaceuticals Inc., Burlingame, CA, USA) and Glucagon-Like Peptide-1 Active ELISA (EMD Millipore, Billerica, MA, USA), respectively. To detect cross-linked reactions, all samples were directly tested at the highest assayed concentration using CCK and GLP-1 ELISA kits prior to assay cell supernatants. All experiments were conducted at least twice using three biological replicates; ELISA measurements were performed in duplicate.

Total RNA was extracted from cultured cells after 10 min incubation with protein digests, using a Nucleospin RNA kit (Macherey-Nagel Gmbh & Co.), according to the manufacturer's instructions. The concentration and purity of each sample were evaluated on a Nanodrop 1000 Spectrophotometer (ThermoFisher Scientific). cDNA was obtained by reverse transcription using a PrimeScript RT Reagent kit (RR037A, TaKaRa Bio Inc., Shiga, Japan). Quantitative RT-PCR amplification was carried out using a real-time thermocycler (Viia 7 Real-Time PCR system; Applied Biosystems, Foster, CA, USA) in 384-well microplates (Axygen, Corning). The SYBR Green method was used and each assay was performed with cDNA samples in triplicate. Amplification was initiated at 50°C for 2 min and at 95°C for 10 min, followed by 40 cycles of 95°C for 15 s and 60 °C for 1 min. The following specific oligonucleotides were used: for CCK (accession no. NM_001284508.2) forward (F) 5'-CCAATTTTTCCTGCCCGCAT-3' (SEQ ID NO: 15) and reverse (R) 5'-AGAAGGAGCAGTCAAGCCAAA-3' (SEQ ID NO: 16); for GLP-1 (accession no. NM_008100.4) (F) 5'-AGAGACATGCTGAAGGGACC-3' (SEQ ID NO: 17) and (R) 5'-CTTTCACCAGCCACGCAATG-3' (SEQ ID NO: 18);14 and for reference gene β-actin (accession no. NM_007393.5): (F) 5'-AGCTGCGTTTTACACCCTTT-3' (SEQ ID NO: 19) and (R) 5'-AAGCCATGCCAATGTTGTCT-3' (SEQ ID NO: 20). The relative expression levels of the target gene were calculated using the comparative critical threshold method (ΔΔCt) by normalizing the data to the expression of β-actin. Experiments were performed at least three times in triplicate.

### a.3.3. Hormone secretion studies in presence of inhibitors

Cells were cultured at 37 °C in a 5% CO₂ humidified atmosphere for 48 h into 24-well plates at a density of 3 × 10⁵ cells per well. Cells were washed twice with HEPES buffer prior to add the inhibitors, NPS2143 (inhibitor of calcium sensing receptor) at 6 µM, TC LPA5 4 (inhibitor of GPR93) at 4 µM or vehicle, dimethyl sulfoxide (DMSO, Sigma-Aldrich) at 1% in HEPES. After 20 min of incubation, synthetic peptides were added into the well (1:1; v:v) at 0.125 mM including the inhibitor at the same concentration to prevent dilution of the inhibitor. Peptides and inhibitors were incubated for 1 h and, after that, supernatants were collected and stored at -80 °C with Halt Protease and phosphatase inhibitor (Thermo Fisher Scientific, Waltham, MA, USA). Measurement of the CCK and GLP-1 concentration was assessed using the commercial ELISA kits. All experiments were conducted at least twice using three biological replicates; ELISA measurements were performed in duplicate.

### Example 1 Secretion of CCK and GLP-1 induced by synthetic peptides derived from egg white and milk proteins in enteroendocrine cells, STC-1

The lysozyme-derived peptides SEQ ID NO: 1 ¹²³WIRGCRL¹²⁹, SEQ ID NO: 2 ¹⁰⁹VAWRNRCKGTD¹¹⁹, and SEQ ID NO: 3 ¹¹¹WRNRCKGTD¹¹⁹ induced a significantly release of both anorexigenic hormones, CCK and GLP-1 (Figure 1 A and B). Remarkably, the levels of GLP-1 in the cell supernatant after the incubation of these peptides at 1 mM reached levels between 884.7 and 1401.5 pM, being these three peptides characterized by a net positive charge. The ovalbumin-derived decapeptide SEQ ID NO: 12 ²⁴⁴VLLPDEVSGL²⁵³ and the related pentapeptide SEQ ID NO: 13 ²⁴⁴VLLPD²⁴⁸, both with a net negative charge, provoked a significant CCK secretion in STC-1 cells. The ovalbumin-derived tripeptide ³⁶⁴PFL³⁶⁶ induced a slightly CCK secretion but a marked GLP-1 secretion. The ovalbumin-derived decapeptide SEQ ID NO: 14 ²¹⁹RVASMASEK²²⁷, with a net positive charge and hydrophobic character, stimulated the release of GLP-1, reaching levels ca. 74 pM, (Figure 1 C and D).

αₛ₁-casein-derived peptides SEQ ID NO: 4 ⁹⁰RYLG⁹³, SEQ ID NO: 5 ⁹⁰RYLGYLE⁹⁶, and SEQ ID NO: 6 ⁹⁰RYLGY⁹⁴; and peptides SEQ ID NO: 7 ³³SRYPS³⁷ and SEQ ID NO: 8 ⁶⁴PAAVRSPAQILQ⁷⁵, derived from κ-casein, induced the secretion of both hormones, CCK and GLP-1 (Figure 2 A and B).

A notable effect was observed for the tetrapeptide SEQ ID NO: 4 ⁹⁰RYLG⁹³ in CCK and GLP-1 secretion and SEQ ID NO: 7 ³³SRYPS³⁷ induced a notable secretion of GLP-1, reaching values of GLP-1 above 650 pM when the peptide was tested at 2 mM. It has to be highlighted that those peptides with a potent GLP-1 secretatogue effect are characterized by a net positive charge (pl ≥ 6) and hydrophobic character, preferably containing arginine and an aromatic residue (W or Y) within their sequences.

Among the β-Lactoglobulin-derived peptides, SEQ ID NO: 10 ⁷³AEKTKIP⁷⁹ induced an important CCK secretion while peptide SEQ ID NO: 9 ¹LlVTQTM⁷ has a highly significant effect on GLP-1 secretion (Figure 2 C and D). This latter peptide had been described as a suppressing appetite peptide (WO2017150536A1) but its effect on the incretin GLP-1, and therefore, on glucose metabolism, had not been previously reported.

**Table 1: Glucagon-like peptide-1 (GLP-1) and cholecystokinin (CCK) levels induced by the synthetic peptides in enteroendocrine cells STC-1**

| **SEQ ID** | **Sequence** | **Precursor protein** | **Peptide assayed concentration (mM)** | **GPL-1 (pM)** | **CCK (pM)** |
|---|---|---|---|---|---|
| SEQ ID NO: 1 | ¹²³WIRGCRL¹²⁹ | LZ P00698 | 1 | 1,401.5* | 133.9* |
| SEQ ID NO: 2 | ¹⁰⁹VAWRNRCKGT0¹¹⁹ | LZ P00698 | 1 | 1,237.9* | 79.0* |
| SEQ ID NO: 3 | ¹¹¹WRNRCKGTD¹¹⁹ | LZ P00698 | 1 | 884.7* | 58.0* |
| SEQ ID NO: 4 | ⁹⁰RYLG⁹³ | αₛ₁-CN P02662 | 2 | 567.1* | 385.5* |
| SEQ ID NO: 5 | ⁹⁰RYLGYLE⁹⁶ | αₛ₁-CN P02662 | 0.5 | 266.4* | 210.1* |
| SEQ ID NO: 6 | ⁹⁰RYLGY⁹⁴ | αₛ₁-CN P02662 | 2 | 113.5* | 127.4* |
| SEQ ID NO: 7 | ³³SRYPS³⁷ | κ-CN P02668 | 2 | 670.7* | 170.6* |
| | | | 0.5 | 187.1* | 128.5* |
| SEQ ID NO: 8 | ⁶⁴PAAVRSPAQILQ⁷⁵ | κ-CN P02668 | 2 | 281.5* | 115.5* |
| SEQ ID NO: 9 | ¹LIVTQTM⁷ | β-Lg P02754 | 1 | 336.7* | 44.0* |
| | | | 0.25 | 108.7* | 25.1* |
| SEQ ID NO: 10 | ⁷³AEKTKIP⁷⁹ | β-Lg P02754 | 2 | 59.0 | 260.7* |
| SEQ ID NO: 11 | ⁷⁸IPAVFK⁸³ | β-Lg P02754 | 2 | 125.0* | 40.9* |
| | | | 0.5 | 49.3* | 20.2* |
| SEQ ID NO: 12 | ²⁴⁴VLLPDEVSGL²⁵³ | OVA P01012 | 2 | 37.8 | 84.7* |
| | | | 0.5 | 40.0 | 69.5* |
| | | | 0.125 | 41.2 | 51.7* |
| SEQ ID NO: 13 | ²⁴⁴VLLPD²⁴⁸ | OVA P01012 | 2 | 39.8 | 69.5* |
| | | | 0.5 | 36.8 | 64.1* |
| | | | 0.125 | 48.2 | 52.4* |
| SEQ ID NO: 14 | ²¹⁹RVASMASEK²² | OVA P01012 | 2 | 73.6* | 35.9 |
| | ³⁶⁴PFL³⁶⁶ | OVA P01012 | 2 | 62.9* | 45.7* |
| | | | 0.5 | 52.3* | 41.5 |
| | | | 0.125 | 43.4 | 34.4 |

| | | | | | |
|---|---|---|---|---|---|
| *Denotes statistically significant differences with the control LZ, lysozyme; κ-CN, κ-casein; β-Lg, β-lactoglobulin; αₛ₁-CN, αₛ₁-casein; OVA, ovalbumin. | | | | | |

### Example 2: Secretion of CCK and GLP-1 induced by egg white hydrolysates in enteroendocrine cells, STC-1

Egg white hydrolysate, prepared with gastrointestinal enzymes as described in Material and methods, was incubated at different concentrations with enteroendocrine cells, STC-1. Figure 3 shows the secretagogue effect of these hydrolysates containing peptides SEQ NO. 1-3, and 12-15 described in Example 1. Egg white hydrolysate induced significant secretion of both hormones, CCK and GLP-1, in a dose-dependent manner (Figure 3 A and B). The levels of GLP-1 in the cell supernatant after the incubation of the digest at different concentrations from 0.125 to 2 mg of protein/mL reached levels between 59 and 200 pM, whereas the CCK level increased up to 471 pM at the highest tested concentration.

After 10 min incubation with egg white hydrolysate, cells were collected and *Cck* and *Glp-1* mRNA levels were evaluated. Egg white hydrolysate at the highest assayed concentration (2 mg/mL) increased significantly *Cck* and *Glp-1* mRNA levels with a maximum fold increase of 1.7 and 2.2, respectively (Figure 4 A and B).

### Example 3: Secretion of CCK and GLP-1 induced by synthetic peptides derived from egg white and milk proteins in presence of G-protein coupled receptor (GPCR)-inhibitors in enteroendocrine cells, STC-1

GPCR-inhibitors, NPS2143 (inhibitor of calcium sensing receptor, CaSR) and TC LPA5 (inhibitor of GPR93), were incubated with the cells for 20 min prior to the addition of synthetic peptides SEQ ID NO: 7 ³³SRYPS³⁷ and SEQ NO: 5 ⁹⁰RYLGYLE⁹⁶ at 0.125 mM (Figure 5 A and B). The CCK and GLP-1 secretagogue effect of SEQ ID NO: 7 ³³SRYPS³⁷ was reduced when co-incubated with both inhibitors (P < 0.01) and this points to the participation of both receptors, CaSR and GPR93, in cell activation. Similarly, both inhibitors partially abolish the CCK secretagogue effect of SEQ ID NO: 5 ⁹⁰RYLGYLE⁹⁶ (198 pM *vs.* 88 pM with NPS2143 and 111 pM with TC LPA5 4), suggesting the involvement of both receptors for the secretion of this hormone. However, the effect of this peptide on GLP-1 secretion is too low to evaluate the effect of the GPCR-inhibitors.

## Claims

1. A peptide resistant to gastrointestinal digestion and capable of inducing anorexic hormone secretion comprising an amino acid sequence selected from sequences SEQ ID NO: 1 to 14 and ³⁶⁴PFL³⁶⁶.

2. The peptide according to claim 1 wherein the anorexic hormone secretion induced is cholecystokinin and/or glucagon-like peptide 1.

3. A composition comprising one or more of the peptides according to claims 1 or 2.

4. Composition according to claim 3, wherein the composition is a food, a beverage or a pharmaceutical composition.

5. A kit comprising the peptide according to claims 1 or 2, or the composition according to claims 3 or 4.

6. The peptide according to claims 1 or 2, or the composition according to claims 3 or 4, or the kit according to claim 5 for use as a medicament.

7. The peptide according to claims 1 or 2, or the composition according to claims 3 or 4, or the kit according to claim 5 for use in the induction of cholecystokinin secretion and/or glucagon-like peptide 1 secretion in a mammal.

8. The peptide according to claims 1 or 2, or the composition according to claims 3 or 4, or the kit according to claim 5 for use in the increase of post-prandial insulin secretion in a mammal.

9. The peptide according to claims 1 or 2, or the composition according to claims 3 or 4, or the kit according to claim 5 for use in lowering plasma blood glucose levels in a mammal.

10. The peptide according to claims 1 or 2, or the composition according to claims 3 or 4, or the kit according to claim 5 for use in lowering liver fat content in a mammal.

11. The peptide according to claims 1 or 2, or the composition according to claims 3 or 4, or the kit according to claim 5 for use in reducing high fat diet or obesity induced inflammation in a mammal.

12. The peptide according to claims 1 or 2, or the composition according to claims 3 or 4, or the kit according to claim 5 for use in regulating glucose homeostasis in a mammal.

13. The peptide according to claims 1 or 2, or the composition according to claims 3 or 4, or the kit according to claim 5 for use in treating and/or preventing hyperglycemia in a mammal.

14. The peptide according to claims 1 or 2, or the composition according to claims 3 or 4, or the kit according to claim 5 for use in treatment or prevention of obesity or overweight in a mammal.
